# EUROPÄISCHE PATENTSCHRIFT

(11) **EP 1 796 882 B1**
(45) Veröffentlichungstag und Bekanntmachung des Hinweises auf die Patenterteilung: **20.08.2008**
(21) Anmeldenummer: 05787094.1
(22) Anmeldetag: 14.09.2005
(51) Int. Cl.: B26B 13/06, A61B 17/32

(54) **SCHERE**
SCISSORS
CISEAUX

(30) Priorität: 20.09.2004 DE 202004014750 U
(43) Veröffentlichungstag der Anmeldung: 20.06.2007
(73) Patentinhaber: Gimmi Gmbh, 78532 Tuttlingen (DE)
(72) Erfinder: MOSER, Klaus, 78576 EMMINGEN-LIPTINGEN (DE)
(74) Vertreter: Patentanwälte Westphal, Mussgnug & Partner
(86) Internationale Anmeldenummer: PCT/EP2005/009874
(87) Internationale Veröffentlichungsnummer: WO 2006/032395

(56) Entgegenhaltungen:
- US-A- 2 568 234
- US-A- 3 721 245
- US-A- 6 079 107

## Beschreibung

Die Erfindung betrifft eine Schere gemäß dem Oberbegriff des Schutzanspruchs 1.

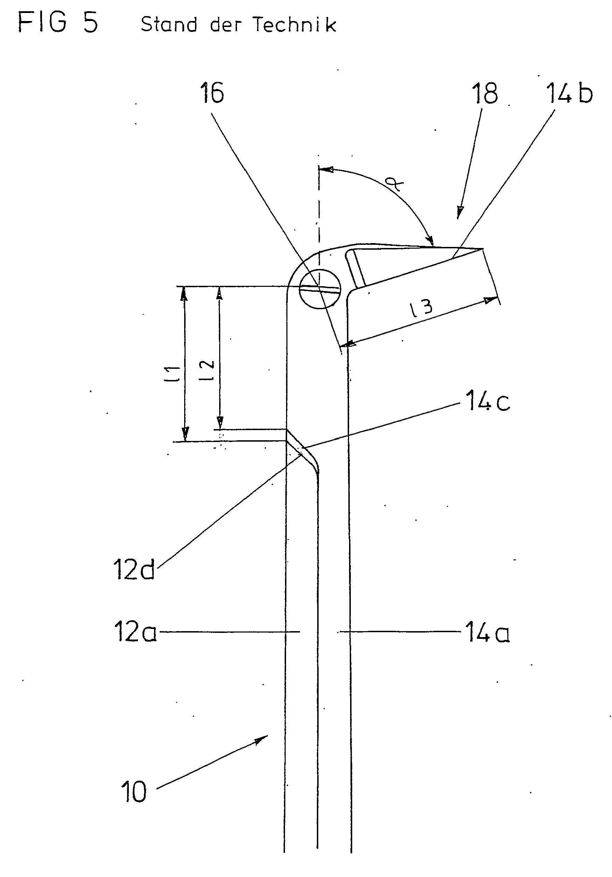

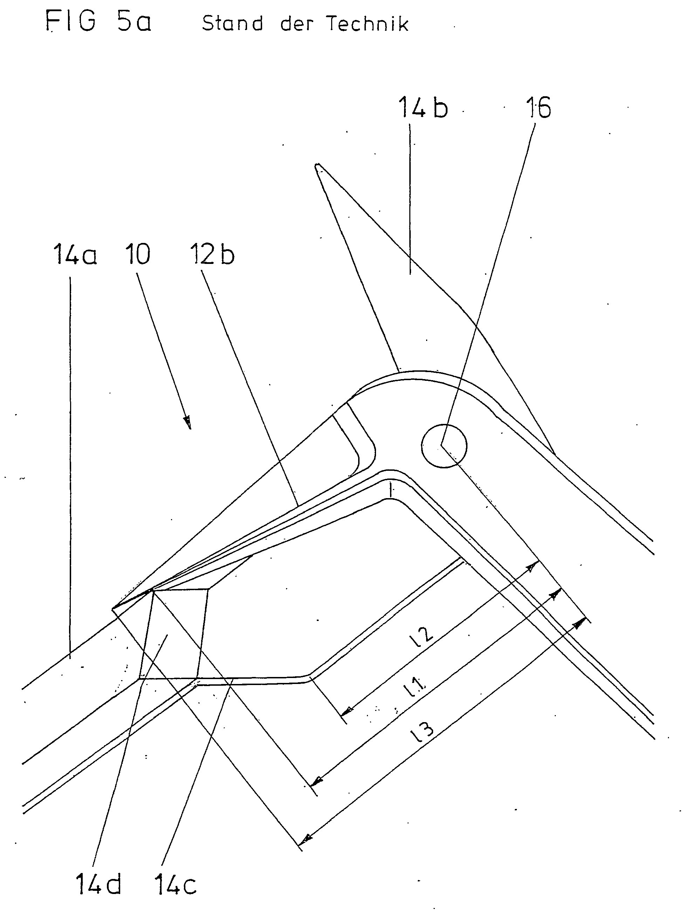

Die Figuren 5 und 5a zeigen eine Schere 10 gemäß dem Stand der Technik mit einem ersten Gelenkteil 12 und einem zweiten Gelenkteil 14, welche um eine Drehachse 16 drehbar gegeneinander gelagert sind. Die Gelenkteile 12, 14 gehen an einem freien axialen Ende über eine Schlussschräge 12d, 14d und eine Gegenfläche 12c, 14c in ein Griffelement 12a, 14a über. An dem anderen freien axialen Ende der Gelenkteile 12, 14 ist ein mit Scherenelementen 12b, 14b ausgestattetes Arbeitsende 18 angeordnet. Werden die beiden Gelenkteile 12, 14 um die Drehachse 16 gedreht und vollständig geschlossen, kommt auf der Schlussschräge 12d des Gelenkteils 12 die Gegenfläche 14c des Gelenkteils 14 und auf der Schlussschräge 14d des Gelenkteils 14 die Gegenfläche 12c des Gelenkteils 13 jeweils nur durch einen sehr kleinen Spalt getrennt zu liegen. Sowohl die Schlussschrägen 12d, 14d als auch die Gegenflächen 12c, 14c verlaufen ausgehend von der Anlagefläche der beiden Gelenkteile 12, 14 schräg gegen die Achse der Schere 10 nach außen und bilden mit dem in Richtung des Arbeitsendes 18 weisenden Abschnitt der Achse der Schere 10 einen spitzen Winkel.

Im Folgenden wird der Abstand L1 zwischen der Drehachse 16 und der Schlussschräge 12d, 14d definiert als der Abstand L1 von der Drehachse 16 zum Schnittpunkt der Schlussschräge 12d, 14d mit der Außenseite des Gelenkteils 12, 14. Der Abstand L2 zwischen der Drehachse 16 und der Gegenfläche 12c, 14c ist definiert als der Abstand L2 von der Drehachse zum Schnittpunkt der Gegenfläche 12c, 14c mit der Außenseite des Gelenkteils 12, 14.

Aus der Chirurgie sind derartige Scheren bekannt, bei welchen das Arbeitsende 18 um einen Winkel α, welcher insbesondere 20° bis 160° beträgt, in der Ebene der Schere 10 von der Achse der Gelenkteile 12, 14 abgewinkelt ist.

Die Scherenelemente 12b, 14b des Arbeitsendes 18 laufen in einem spitzen distalen Ende aus und sind somit besonders empfindlich. Bei Belastung brechen diese distalen Enden der Scherenelemente 12b, 14b leicht ab. In Figur 5a ist eine derartige Belastungssituation dargestellt, welche auftreten kann, wenn die beiden Gelenkteile 12, 14 besonders weit geöffnet werden, sodass das Scherenelement 12b des ersten Gelenkteils 12 auf das Griffelement 14a des zweiten Gelenkteils 14 aufschlägt.

Eine weitere derartige Schere ist auch aus US 3,721,245 bekannt.

Die Aufgabe der Erfindung liegt darin, eine Schere bereit zu stellen, bei welcher die distalen Enden vor unnötiger Belastung, welche zu Zerstörung führen kann, geschützt werden.

Die Aufgabe wird erfindungsgemäß gelöst durch eine Schere mit den Merkmalen des kennzeichnenden Teils des Schutzanspruchs 1.

Vorteilhafte Weiterbildungen der Erfindung sind in den Unteransprüchen angegeben.

Erfindungsgemäß ist der Abstand von der Drehachse zur Schlussschräge wenigstens eines der Gelenkteile größer als der Abstand von der Drehachse zum distalen Ende der Scherenelemente.

Dies bedingt, dass auch bei großem Öffnungswinkel der beiden Gelenkteile gegeneinander das distale Ende des Scherenelements des ersten Gelenkteils nicht auf dem Griffelement des anderen Gelenkteils aufschlagen kann, sondern daran vorbei drehen kann. Die empfindlichen distalen Enden der Scherenelemente sind somit vor einem Abbrechen durch zu weites Öffnen der Schere und Aufschlagen auf dem Griffelement des anderen Gelenkteils geschützt.

Vorzugsweise ist der Abstand der Drehachse zur Schlussschräge bei beiden Gelenkteilen größer als der Abstand von der Drehachse zum distalen Ende der Scherenelemente, um beide Scherenelemente entsprechend zu schützen.

Bei einer vorteilhaften Weiterbildung der Erfindung ist der Abstand von der Drehachse zu einer Gegenfläche des Griffelements kleiner als der Abstand von der Drehachse zur Schlussschräge. Eine derartige Ausgestaltung kommt insbesondere dann zur Anwendung, wenn zur Herstellung der Gelenkteile normierte Rohlinge gemäß dem Stand der Technik verwendet werden, deren Abstand von der Drehachse zur Schlussschräge und somit ebenfalls zur Gegenfläche kleiner ist als der Abstand von der Drehachse zum distalen Ende der Scherenelemente. Um derartige Rohlinge zur Herstellung der erfindungsgemäßen Schere verwenden zu können, werden die Schlussschrägen parallel zum proximalen Ende der Gelenkteile hin versetzt, indem entsprechend Material von den Rohlingen entfernt wird. Die Schlussschrägen werden um einen bestimmten Betrag soweit parallel zum proximalen Ende der Gelenkteile versetzt, bis der Abstand der Drehachse zur Schlussschräge größer ist als der Abstand von der Drehachse zum distalen Ende des Scherenelements. In diesem Fall verbleibt jedoch der Abstand von der Drehachse zur Gegenfläche des Griffelements um den bestimmten Betrag kleiner als der Abstand von der Drehachse zur Schlussschräge. Auf diese Weise können besonders einfach bekannte Rohlinge zur Herstellung der erfindungsgemäßen Schere verwendet werden.

In einer alternativen bevorzugten Ausführungsvariante der Erfindung entspricht der Abstand von der Drehachse zu der Gegenfläche in etwa dem Abstand von der Drehachse zur Schlussschräge der Gelenkteile. Somit sind beide Abstände größer als der Abstand von der Drehachse zum distalen Ende der Scherenelements. Zur Herstellung können keine Rohlinge gemäß dem Stand der Technik verwendet werden, dafür liegen jedoch im geschlossenen Zustand der Schere die Gegenflächen jeweils auf den Schlussschrägen auf.

Bevorzugt beträgt der Winkel, um welchen das Arbeitsende abgewinkelt ist, 20° bis 160°. Besonders bevorzugt ist das Arbeitsende um 90° bis 135° in der Ebene der Schere von der Achse der Gelenkteile abgewinkelt. Bei kleineren Winkeln tritt das Problem einer Beschädigung der distalen Enden der Scherenelemente nur sehr selten auf, da dafür ein besonders großer Öffnungswinkel der beiden Gelenkteile von Nöten wäre, der jedoch sehr selten erreicht wird.

Die Erfindung wird anhand der in den folgenden Figuren gezeigten Ausführungsbeispiele ausführlich erläutert. Es zeigt
- Fig. 1: ein erstes Ausführungsbeispiel einer erfindungsgemäßen Schere,
- Fig. 1a: die Schere aus Fig. 1 in geöffnetem Zustand,
- Fig. 2: ein zweites Ausführungsbeispiel der Erfindung,
- Fig. 3: ein drittes Ausführungsbeispiel der Erfindung,
- Fig. 4: ein viertes Ausführungsbeispiel der Erfindung,
- Fig. 5: ein Ausführungsbeispiel einer Schere gemäß dem Stand der Technik und
- Fig. 5a: die Schere gemäß Fig. 5 in geöffnetem Zustand.

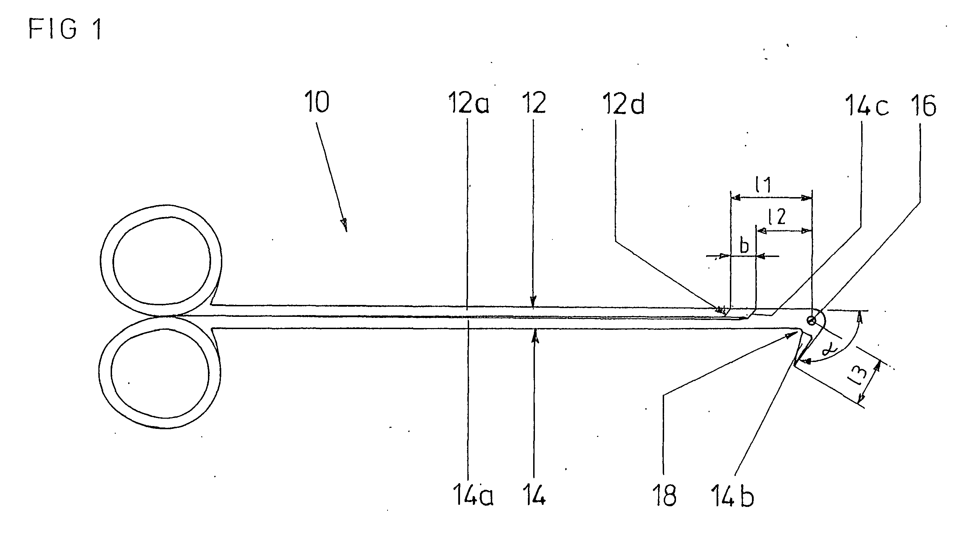

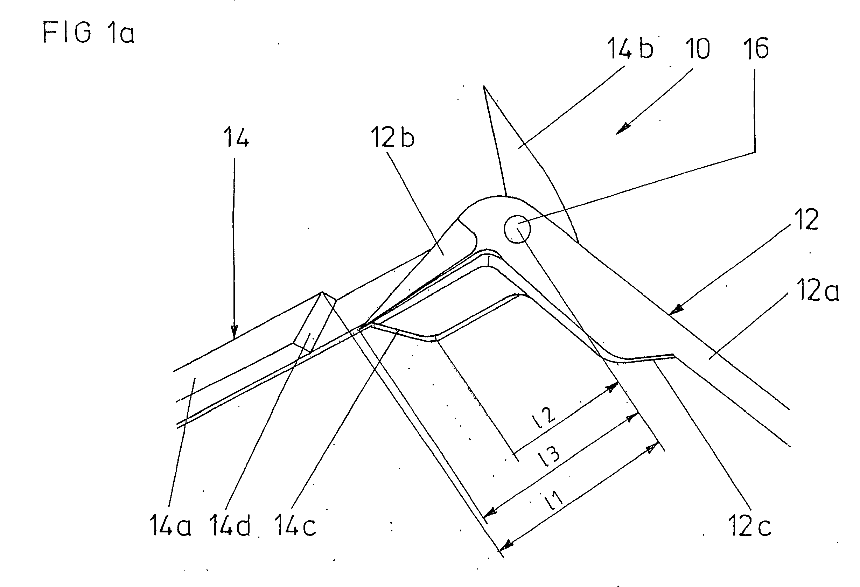

Figur 1 zeigt ein erstes Ausführungsbeispiel einer erfindungsgemäßen Schere 10 mit einem ersten Gelenkteil 12 und einem zweiten Gelenkteil 14, welche über eine Drehachse 16 drehbar gegeneinander gelagert sind. Die beiden Gelenkteile 12, 14 weisen an einem freien axialen Ende auf einer Seite der Drehachse 16 jeweils ein Scherenelement 12b, 14b auf, welche gemeinsam ein Arbeitsende 18 bilden. Das Arbeitsende 18 ist dabei um einen Winkel α, welcher vorliegend etwa 125° beträgt, in der Ebene der Schere 10 von der Achse der Gelenkteile 12, 14 abgewinkelt. Die freien axialen Enden der Gelenkteile 12, 14 auf der anderen Seite der Drehachse 16 gehen über eine Gegenfläche 12c, 14c in ein Griffelement 12a, 14a über. Die Griffelemente 12a, 14a sind dabei nach Art der Griffelemente bekannter Scheren ausgebildet.

Die Gelenkteile 12, 14 weisen jeweils eine Schlussschräge 12d, 14d auf, welche ausgehend von der Gegenfläche 14c, 12c des jeweils anderen Gelenkteils 14, 12 um den Betrag b entlang der Achse der Gelenkteile 12, 14 zum proximalen Ende der Schere 10 parallel versetzt sind. Dazu wurde ausgehend von einem Rohling, bei welchem die Schlussschrägen 12d, 14d direkt an den Gegenflächen 12c, 14c zu liegen kommen (vgl. bspw. Figuren 5 und 5a), Material entfernt, um die Schlussschrägen 12d, 14d parallel zum proximalen Ende der Gelenkteile 12, 14 hin zu versetzen. Durch dieses Versetzen der Schlussschrägen 12d, 14d wird der Abstand L1 von der Drehachse 16 zu den Schlussschrägen 12d, 14d größer als der Abstand L3 von der Drehachse 16 zu dem distalen Ende der Scherenelemente 12b, 14b. Der Abstand L2 von der Drehachse 16 zu den Gegenflächen 12c, 14c verbleibt in diesem Ausführungsbeispiel der Erfindung kleiner als der Abstand L3 von der Drehachse 16 zu den distalen Enden der Scherenelemente 12b, 14b (vgl. Figur 1a).

Der Vorteil eines derartigen Versatzes der Schlussschrägen 12d, 14d zeigt sich in Figur 1a, in welcher die erfindungsgemäße Schere 10 mit geöffneten Gelenkteilen 12, 14 dargestellt ist. Dadurch, dass der Abstand L1 von der Schlussschräge 14d zu der Drehachse 16 größer ist als der Abstand L3 von der Drehachse 16 zu dem distalen Ende des Scherenelements 12b, lässt sich das Gelenkteil 12 mit dem Scherenelement 12b weit öffnen, ohne dass das distale Ende des Scherenelements 12b auf dem Griffelement 14a des Gelenkteils 14 aufschlägt und die Gefahr des Abbrechens des distalen Endes des Scherenelements 12b bestehen könnte, da das distale Ende des Scherenelements 12b an dem Griffelement 14a des Gelenkteils 14 vorbeidrehen kann.

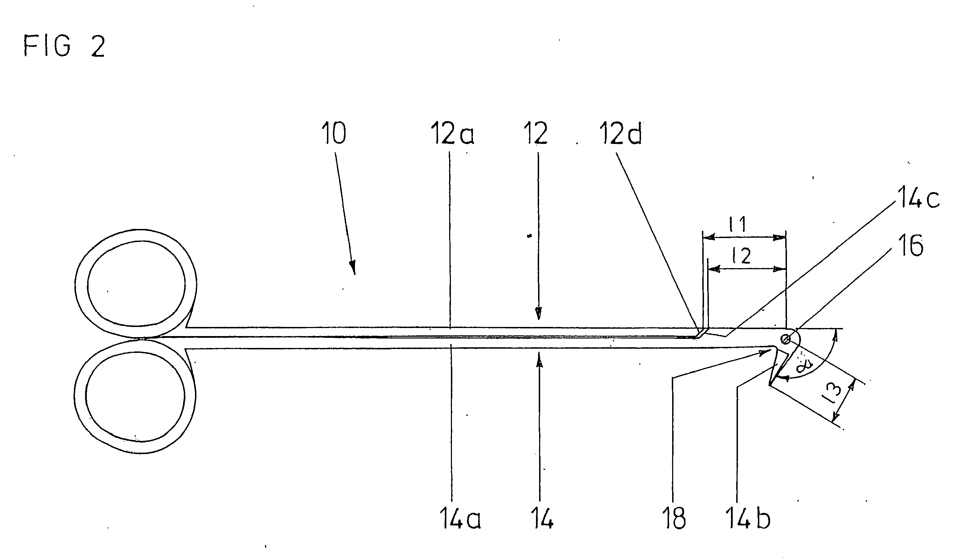

Figur 2 zeigt ein weiteres Ausführungsbeispiel der Erfindung, bei welchem gleiche Teile wie im ersten Ausführungsbeispiel mit gleichen Bezugsziffern bezeichnet sind. Das zweite Ausführungsbeispiel unterscheidet sich von dem Ausführungsbeispiel in Figur 1 nur-dadurch, dass auch der Abstand L2 von der Gegenfläche 14c zur Drehachse 16 größer ist als der Abstand L3 von der Drehachse 16 zum distalen Ende der Scherenelemente 12b, 14b. Der Abstand L2 ist in etwa so groß wie der Abstand L1, sodass die Gegenfläche 14c auf der Schlussschräge 12d nur durch einen sehr kleinen Spalt getrennt zu liegen kommt. Eine derartige Schere 10 kann nicht aus Rohlingen hergestellt werden, bei welchen die Abstände L1 und L2 kleiner sind als die Abstände L3, da zusätzlich zum Versatz der Schlussschrägen 12d, 14d, für welche Material entfernt werden muss, auch die Gegenflächen 12c, 14c zum proximalen Ende versetzt werden, wofür Material hinzugefügt werden müsste.

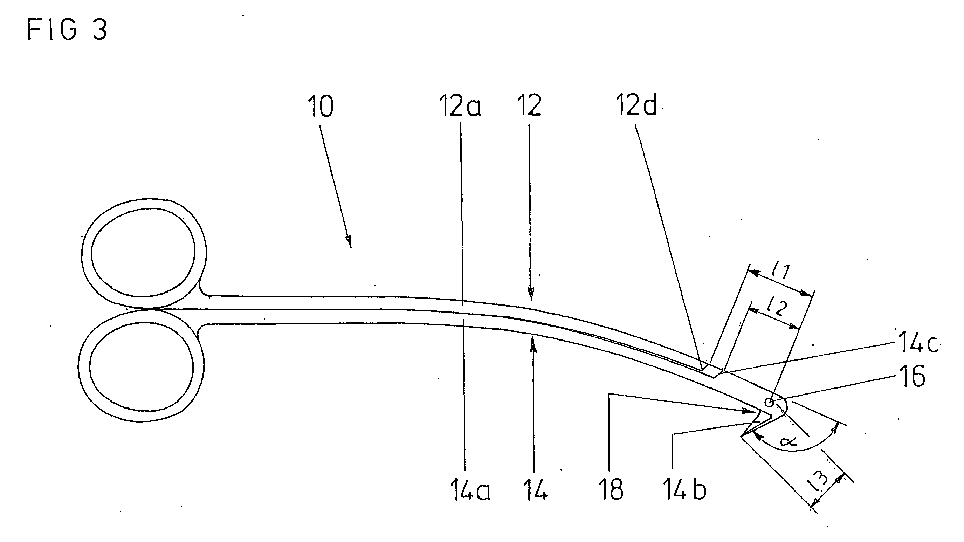

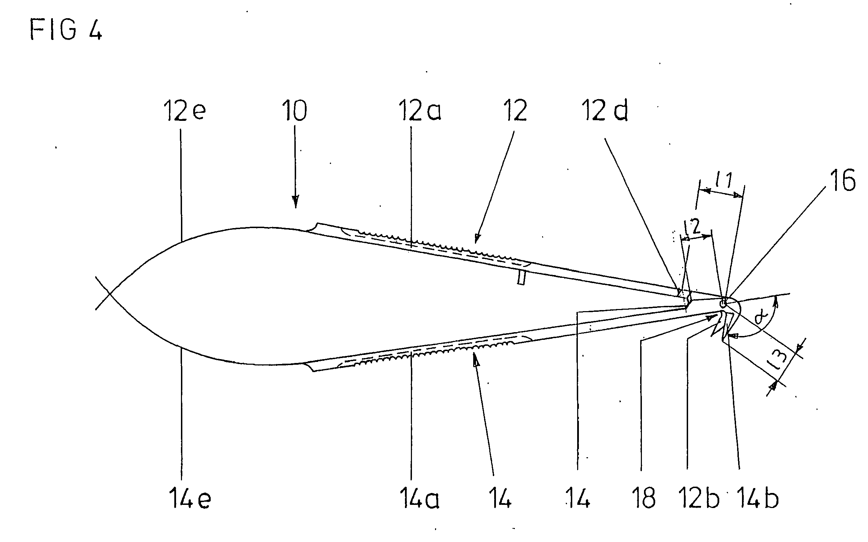

Die Figuren 3 und 4 zeigen zwei weitere Ausführungsbeispiele der Erfindung, welche sich von dem in der Figur 1 gezeigten Ausführungsbeispiel nur durch die Ausgestaltung der Griffelemente 12a, 14a unterscheiden. Das in Figur 3 gezeigte Ausführungsbeispiel weist Griffelemente 12a, 14a auf, welche leicht gebogen sind, um je nach Operationsraum das Arbeitsende 18 besonders leicht einführen zu können. Das in Figur 4 gezeigte Arbeitsende weist zwei Griffelemente 12a, 14a auf, welche über zwei Federelemente 12e, 14e in geöffnetem Zustand gehalten werden, sodass die Schere des Arbeitsendes 18 gegen die Kraft der Federelemente 12e, 14e durch Zusammendrücken der Griffelemente 12a, 14a geschlossen werden kann.

### Bezugszeichenliste

- 10: Schere
- 12: erstes Gelenkteil
- 12a: Griffelement
- 12b: Scherenelement
- 12c: Gegenfläche
- 12d: Schlussschräge
- 12e: Federelement
- 14: zweites Gelenkteil
- 14a: Griffelement
- 14b: Scherenelement
- 14c: Gegenfläche
- 14d: Schlussschräge
- 14e: Federelement
- 16: Drehachse
- 18: Arbeitsende

- L1: Abstand
- L2: Abstand
- L3: Abstand

- α: Winkel

- b: Betrag

## Patentansprüche

1. Schere mit zwei Gelenkteilen (12, 14), welche um eine Drehachse (16) drehbar gegeneinander gelagert sind, welche an einem freien axialen Ende über eine Schlussschräge (12d, 14d) und eine Gegenfläche (12c, 14c) in ein Griffelement (12a, 14a) übergehen und welche an dem anderen freien axialen Ende ein mit Scherenelementen (12b, 14b) ausgestattetes Arbeitsende (18) aufweisen, wobei das Arbeitsende (18) um einen Winkel (α) in der Ebene der Schere (10) von der Achse der Gelenkteile (12, 14) abgewinkelt ist,
**dadurch gekennzeichnet, dass**
der Abstand (L1) von der Drehachse (16) zur Schlussschräge (12d, 14d) wenigstens eines der Gelenkteile (12, 14) größer ist als der Abstand (13) von der Drehachse (16) zum distalen Ende der Scherenelemente (12b, 14b).

2. Schere nach Anspruch 1,
**dadurch gekennzeichnet, dass**
der Abstand (L1) der Drehachse (16) zur Schlussschräge (12d, 14d) bei beiden Gelenkteilen (12, 14) größer ist als der Abstand (13) von der Drehachse (16) zum distalen Ende der Scherenelemente (12b, 14b).

3. Schere nach Anspruch 1 oder 2,
**dadurch gekennzeichnet, dass**
der Abstand (L2) von der Drehachse (16) zu der Gegenfläche (12c, 14c) um einen Betrag (b) kleiner ist als der Abstand (11) von der Drehachse (16) zur Schlussschräge (12d, 14d).

4. Schere nach Anspruch 1 oder 2,
**dadurch gekennzeichnet, dass**
der Abstand (L2) von der Drehachse (16) zu der Gegenfläche (12c, 14c) in etwa dem Abstand (L1) von der Drehachse (16) zur Schlussschräge (12d, 14d) entspricht.

5. Schere nach einem der vorhergehenden Ansprüche,
**dadurch gekennzeichnet, dass**
der Winkel (α) etwa 20° bis 160° beträgt.

6. Schere nach einem der vorhergehenden Ansprüche,
**dadurchgekennzeichnet,** dass
der Winkel (α) etwa 90° bis 135° beträgt.

## Claims

1. Scissors having two jointed parts (12, 14), which are mounted for mutual rotation around an axis of rotation (16), which at a free axial end make the transition into a grip element (12a, 14a) via a stop inclined edge (12d, 14d) and a counter-surface (12c, 14c) and which at the other free axial end comprise an operative end (18) equipped with scissors elements (12b, 14b), the operative end (18) being bent by an angle (α) in the plane of the scissors (10) from the axis of the jointed parts (12, 14),
**characterised in that** the distance (11) from the axis of rotation (16)) to the stop inclined edge (12d, 14d) of at least one of the jointed parts (12, 14) is greater than the distance (13) from the axis of rotation (16) to the distal end of the scissors elements (12b, 14b).

2. Scissors according to Claim 1,
**characterised in that** the distance (11) from the axis of rotation (16) to the stop inclined edge (12d, 14d) for the two jointed parts (12, 14) is greater than the distance (13) from the axis of rotation (16) to the distal end of the scissors elements (12b, 14b).

3. Scissors according to Claim 1 or 2,
**characterised in that** the distance (12) from the axis of rotation (16) to the counter-surface (12c, 14c) is smaller by an amount (b) than the distance (11) from the axis of rotation (16) to the stop inclined edge (12d, 14d).

4. Scissors according to Claim 1 or 2,
**characterised in that** the distance (12) from the axis of rotation (16) to the counter-surface (12c, 14c) corresponds roughly to the distance (11) from the axis of rotation (16) to the stop inclined edge (12d, 14d).

5. Scissors according to one of the preceding Claims,
**characterised in that** the angle (α) is roughly 20° to 160°

6. Scissors according to one of the preceding Claims,
**characterised in that** the angle (α) is roughly 90° to 135°

## Revendications

1. Ciseaux comportant deux pièces articulées (12, 14) montées pivotantes l'une par rapport à l'autre autour d'un axe de rotation (16) et qui rejoignent leur extrémité axiale libre, par l'intermédiaire d'une rampe de terminaison (12d, 14d) et d'une surface antagoniste (12c, 14c), un élément de préhension (12a, 14a) et qui à l'autre extrémité axiale libre comporte une extrémité de travail (18) équipée d'éléments de ciseaux (12b, 14b),
l'extrémité de travail (18) étant repliée d'un angle (α) dans le plan des ciseaux (10) par rapport à l'axe des pièces d'articulation (12, 14),
**caractérisés en ce que**
la distance (L1) entre l'axe de rotation (16) et les rampes de terminaison (12d, 14d) d'au moins l'une des pièces d'articulation (12, 14) est supérieure à la distance (13) entre l'axe de rotation (16) et l'extrémité distale des éléments de ciseaux (12b, 14b).

2. Ciseaux selon la revendication 1,
**caractérisés en ce que**
la distance (L1) de l'axe de rotation (16) par rapport aux rampes de terminaison (12d, 14d) pour les deux pièces articulées (12, 14) est supérieure à la distance (13) entre l'axe de rotation (16) et l'extrémité distale des éléments de ciseaux (12b, 14b).

3. Ciseaux selon les revendications 1 ou 2,
**caractérisés en ce que**
la distance (L2) entre l'axe de rotation (16) et la surface antagoniste (12c, 14c) est inférieure d'une distance (b) à la distance (11) entre l'axe de rotation (16) et la rampe de terminaison (12d, 14d).

4. Ciseaux selon les revendications 1 ou 2,
**caractérisés en ce que**
la distance (L2) entre l'axe de rotation (16) et la surface antagoniste (12c, 14c) correspond sensiblement à la distance (L1) entre l'axe de rotation (16) et la rampe de terminaison (12d, 14d).

5. Ciseaux selon l'une des revendications précédentes,
**caractérisés en ce que**
l'angle (α) est d'environ 20° à 160°.

6. Ciseaux selon l'une des revendications précédentes,
**caractérisés en ce que**
l'angle (α) est compris entre environ 90° et 135°.
